# EUROPEAN PATENT SPECIFICATION

(11) **EP 4 262 966 B1**
(45) Date of publication and mention of the grant of the patent: **05.08.2026**
(21) Application number: 21840679.1
(22) Date of filing: 16.12.2021
(51) Int. Cl.: A61N 1/04, A61M 21/02, A61N 1/36, A61M 21/00, A61B 5/28, A61B 5/00, G16H 20/30, G16H 40/63

(54) **DEVICE AND SYSTEM TO INDUCE FALLING ASLEEP**
VORRICHTUNG UND SYSTEM ZUR AUSLÖSUNG DES EINSCHLAFENS
DISPOSITIF ET SYSTÈME POUR INDUIRE L'ENDORMISSEMENT

(30) Priority: 18.12.2020 IT 202000031376
(43) Date of publication of application: 25.10.2023
(73) Proprietor: Neuroconnect S.r.l., 00168 Roma (RM) (IT); Bionen S.r.l., 50127 Firenze (FI) (IT)
(72) Inventor: VECCHIO, Fabrizio, 00175 Roma RM (IT); MIRAGLIA, Francesca, 85100 Potenza PZ (IT); DE GENNARO, Luigi, 00141 Roma RM (IT); ROSSINI, Paolo Maria, 00153 Roma RM (IT); TIRELLI, Simone, 50137 Firenze FI (IT)
(74) Representative: Papa, Elisabetta
(86) International application number: PCT/IB2021/061855
(87) International publication number: WO 2022/130276

(56) References cited:
- EP-A2- 2 524 649
- WO-A2-2009/044271
- US-A1- 2016 346 530
- US-A1- 2018 310 855
- US-A1- 2019 030 336
- US-A1- 2019 134 395
- US-A1- 2019 282 812
- US-A1- 2019 321 583
- US-A1- 2020 086 078
- US-A9- 2020 155 790
- D'ATRI A ET AL: "Electrical stimulation of the frontal cortex enhances slow-frequency EEG activity and sleepiness", NEUROSCIENCE, NEW YORK, NY, US, vol. 324, 8 March 2016 (2016-03-08), pages 119 - 130, XP029511937, ISSN: 0306-4522, DOI: 10.1016/J.NEUROSCIENCE.2016.03.007

## Description

### Technical field of the invention

The present invention mainly refers to a device and a system for inducing falling asleep in a subject. The invention is based on personalized electrostimulation of the brain by means of a plurality of electrodes applied to the scalp.

### Background

From an electrophysiological point of view, brain functioning is determined by the endogenous bioelectric activity of the nerve cells, which is represented by electroencephalographic traces. The electroencephalogram (EEG) represents the oscillatory signals recorded by electrodes positioned on the scalp that contain characteristics in frequency (c/sec or Hertz) and in topography (frontal, temporal, parietal lobe, etc.).

The oscillations are defined with letters of the Greek alphabet (delta = 0.5-4 Hz; theta = 4-8 Hz; alpha = 8-12 Hz; beta = 13-20 Hz; gamma = 20-100 Hz) and vary in their representation as a function of the state of the brain that produces them (e.g. wakefulness, sleep state and its various phases, cognitive activity, mental rest, etc.).

It has been scientifically proven that the characteristics of the EEG frequencies over time and space have a precise meaning in the physiology of the brain and of the whole organism; for example, they reflect various states of wakefulness and sleep, as well as phases of learning or performing specific tasks.

Transcranial electrical stimulation of the brain with oscillatory alternating polarity (i.e. similar to EEG oscillations) performed by means of dispensing electrodes - once the "resistance" of the skull and extracerebral envelopes (meninges, liquor *et al*) has been overcome - is capable of inducng a perturbation and/or modulation of the spontaneous electroencephalic activity and in particular of the rhythms according to which it oscillates over time. In other words it is possible through the so-called tACS (*transcranial Alternating Current Stimulation)* to progressively "drive" the oscillatory EEG activity of the brain towards the same frequency and phase characteristics of the stimulus.

The methods of electrical (or magnetic) stimulation are potentially preferable to the pharmacological ones inherent to brain functions, because they are more specific and selective than a drug that must be distributed throughout the body to reach the brain.

Transcranial electrical stimulation can be performed in direct current (*transcranial direct current stimulation,* tDCS) or alternating current (transcranial *alternating current stimulation,* tACS). These two types of current delivery act through different neurobiological mechanisms. In fact, the tDCS hyperpolarizes / depolarizes the stimulated "clod" of cortical neurons, facilitating or reducing the excitability of the corresponding area and the circuits connected to it. The tACS, as described above, can directly modulate the cerebral oscillatory activity represented by the underlying EEG rhythms and the physiological and cognitive processes related to them.

In a recent study [D'Atri A., De Simoni E., Gorgoni M., Ferrara M., Ferlazzo F., Rossini P.M., De Gennaro L.: *Frequency-dependent effects of oscillatory-tDCS on EEG oscillations: a study with better oscillation detection method (BOSC);* Archives Italiennes de Biologie 153(2-3):134-44] the electroencephalographic effects of oscillatory tDCS were investigated in order to detect the changes induced within the specific spontaneous oscillatory electroencephalic activity.

In a subsequent experiment, the possibility of using oscillatory stimulation in order to artificially induce an electroencephalic activity compatible with sleep was explored [D'Atri A., De Simoni E., Gorgoni M., Ferrara M., Ferlazzo F., Rossini P.M., De Gennaro L.: Electrical stimulation of the frontal cortex enhances slow-frequency EEG activity and sleepiness; Neuroscience 2016; 324:119-130], observing an increase in delta activity - typical of falling asleep and sleep - in frontal areas following anode stimulation at 5 Hz, in parallel with an increased perception of subjective sleepiness.

Also known in the art are multiple devices and systems for acquisition or transcranial electrical stimulation. US2019/0030336 discloses a wearable device and a transcranial electrical stimulation method based on pre-stored external data.

US2020/0086078 describes a method for recording a desired state of mental excitement of a first donor subject and replicating it in a second recipient subject. However, to date the documents of known art and the theoretical studies carried out have not led to the development of non-invasive devices and systems that are practically usable by a subject to promote spontaneous falling asleep. In particular, the devices present in the prior art are unable to perform both the acquisition of electroencephalographic signals of a subject and personalized transcranial electrostimulation based on the recorded electroencephalographic signals of the same.

### Summary of invention

The technical problem posed and solved by the present invention is therefore to provide a device and/or a system that allow to meet the need mentioned above with reference to the prior art.

This problem is solved by a device according to claim 1.

On the basis of a further aspect of the invention, a system according to claim 13 is provided.

Preferred features of the present invention are the subject of the dependent claims.

The method disclosed herein does not form part of the claimed invention.

The invention provides a device and a system to induce
falling asleep. The system operates through an acquisition/recording of the individual electroencephalographic signal during physiological falling asleep, its processing for the extraction of the individual characteristics of the distribution over time and in topography of the various oscillatory frequencies of the EEG and a subsequent electrostimulation with one or more external electrodes that reproduce the same space-time EEG pattern of falling asleep previously "learned" by the system on that specific subject (personalization of the treatment).

The proposed stimulation is therefore configured to "guide" the underlying electroencephalographic rhythms in such a way as to reproduce, or approximate, on an individual level the same trend in space and time of the characteristics of the physiological trend of the electroencephalographic signal during the falling asleep phase.

In one aspect, the invention is based on a dynamic electrostimulation mode, preferably at variable frequency, of selected areas of the brain, performed from the outside using one or more stimulation electrodes positioned in various points of the scalp. Advantageously, the stimulation is completely safe, not disturbing because it is performed with very low intensity current and is of the tACS type (*transcranial Alternating Current Stimulation*).

The brain areas (i.e. the topology), the frequency (s) and the duration for each frequency, the amplitude and / or intensity of stimulation can be evaluated for each subject through an algorithm based on the analysis of the electroencephalographic signal acquired during the 'physiological falling asleep and on the recognition of the parameters associated with the latter.

In one of its embodiments, the system of the invention is able to acquire one or more EEG traces of the subject and, subsequently, to stimulate the brain through a personalized pattern of signals, in particular at specific frequencies and on specific areas of the scalp, which change in the minutes before falling asleep.

Advantageously, the acquisition and/or stimulation phase are performed by means of a wearable device, preferably in the shape of a helmet.

The system typically comprises hardware and software means for carrying out said acquisition of signals, an analysis and processing of the latter and therefore the aforementioned stimulation based on the processed signals.

In one embodiment thereof, the system also includes means of filing of the EEG traces acquired. In a preferred configuration, the aforementioned hardware and software means may include a remote control or mobile communication device, for example a *smart phone* or *tablet,* in particular for the remote control of the acquisition and processing of signals through a dedicated "app".

The general concept of a device capable of recording and processing the EEG signal trend in a given condition and then reproducing it using a tACS stimulation method can be extended to other clinical and non-clinical "personalized" stimulation situations. The device and the general architecture of the system proposed here, therefore, can be applied to other pathologies or clinical conditions other than sleep disorders.

Other advantages, characteristics and methods of use of the present invention will become evident from the following detailed description of some embodiments, presented by way of example and not of limitation.

### Brief description of figures

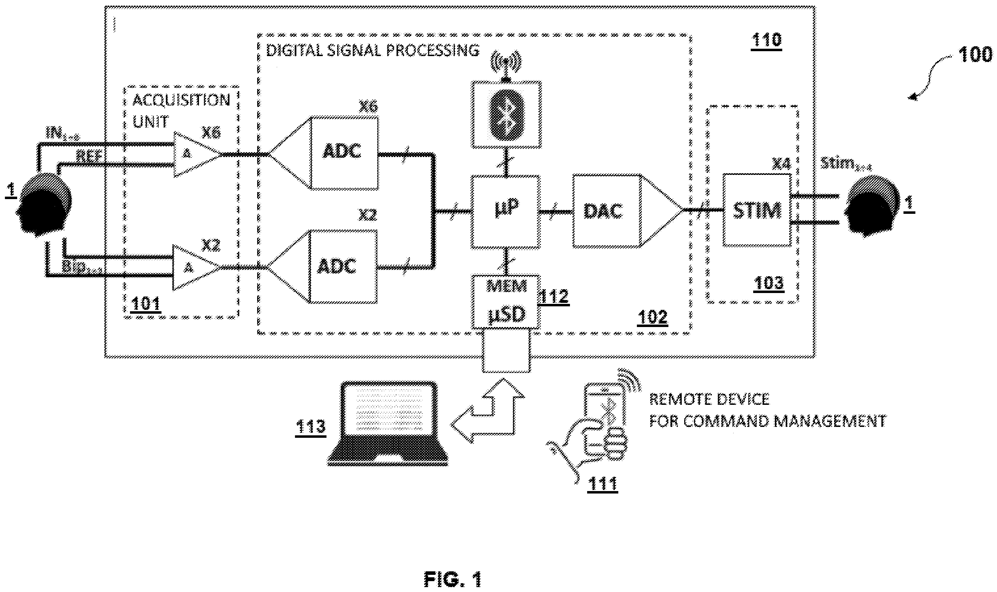

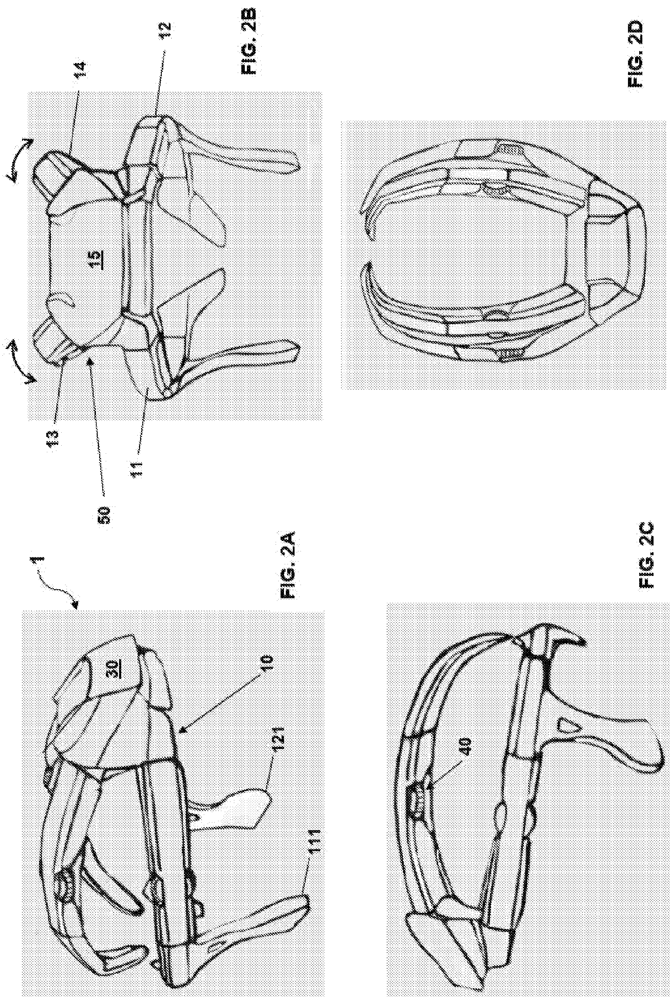

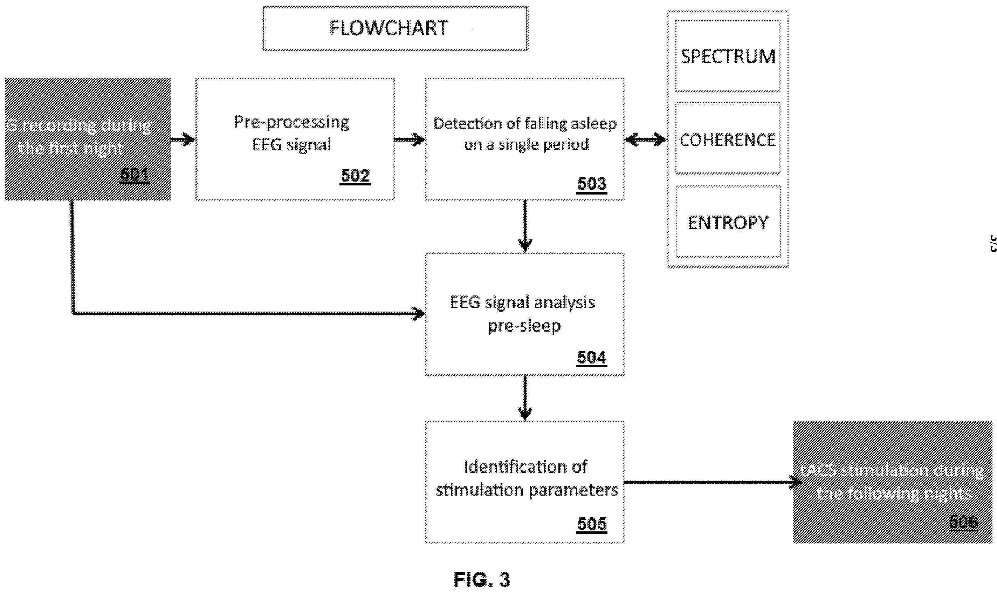

Reference will be made to the figures of the attached drawings, in which:
▪ Figure 1 shows a schematic and exemplary representation in the form of a block diagram of a stimulation system to induce falling asleep according to a preferred embodiment of the present invention;
▪ Figures 2A to 2D refer to an embodiment of a stimulation device that can be included in the system of Figure 1, showing respectively a front perspective view, a front view, a side view and a top plan view;
▪ Figure 3 shows a flow chart relating to an embodiment of a sleep stimulation method that can be implemented using the system of Figure 1.

### Detailed description of preferred embodiments

Various embodiments and variants of the invention will be described below, and this with reference to the figures introduced above.

In the detailed description that follows, further embodiments and variants with respect to embodiments and variants already treated in the same description will be illustrated limitedly to the differences with what has already been disclosed.

Furthermore, the different embodiments and variants described below are capable of being used in combination, where compatible.

With reference initially to Figure 1, a sleep stimulation according to a preferred embodiment of the invention is generally denoted with 100.

The device 100 mainly comprises:
▪ an electroencephalographic signal acquisition unit 101, including, or in communication with, one or more acquisition electrodes, in particular up to 8 simultaneous channels that explore as many brain areas;
▪ a unit 102 for processing said acquired electroencephalographic signals, configured to analyze the latter and to output electroencephalic stimulation signals;
▪ an electroencephalic stimulation unit 103, including, or in communication with, one or more stimulation electrodes applied to the subject's scalp at least in correspondence with the frontal, central and parietal regions of the latter.

The aforementioned units are in operational data communication with each other and, preferably, at least each with the next. Furthermore, they are controlled and / or commanded by a control unit or means 110. The latter are identified by way of example in Figure 1 and may consist of various hardware and software elements also widespread in the system 100.

In general, the aforementioned units and means can be physically located, in whole or in part, on different hardware components, even remote from each other. For example, at least part of the control unit 110 and the analysis and processing unit 102 can be configured on one or more mobile communication devices, in particular one or more tablets or smart phones, in the availability of a healthcare worker and/or of the subject to be stimulated. In the example shown, the start and end control of the acquisition and/or the start and end of stimulation is performed using a smart phone 111, in particular through a dedicated application ("app"). This same device 111, or different means, can be used to receive/provide useful information during the acquisition and / or the stimulation treatment.

Advantageously, the same electrodes can be used for both acquisition and stimulation.

In a particularly preferred embodiment, the electrodes are housed, in a fixed or removable way, on a stimulation device 1, preferably wearable and even more preferably in the form of a helmet. Advantageously, the same device 1 can be configured both for the signal acquisition phase and for the stimulation phase.

A preferred embodiment of the aforementioned wearable device 1 will be described later with reference to Figures 2A-2D.

The hardware and software components of the system 100 of the embodiment of Figure 1 will now be described in greater detail, also with reference to the relative operating modes as schematized in the flow diagram of Figure 3.

Following a specific manual or programmed command, the system 100, through the unit 101, can acquire the electroencephalographic signals of the subject during a physiological fall asleep, for example through eight independent channels, as exemplified in Figure 1 and in phase 501 of Figure 3. In the example considered here, six acquisition channels (IN₁₋₆) are monopolar and preferably associated with contact points, i.e. acquisition electrodes, placed on the frontal, central and parietal scalp areas. Again in the example considered, two acquisition channels (Bip₁₋₂) are bipolar and preferably associated with acquisition electrodes arranged on the chin and the periocular area. As shown in Figure 1, two electrodes for capturing a reference signal (REF) are also provided.

These acquired signals are converted (block "ADC" *- Analog Digital Converter -* in Figure 1), amplified and filtered in the processing unit 102 (block *"digital signal processing"* including a control microprocessor µP in Figure 1, step 502 of *"pre -processing* "in Figure 3).

The sampling of the electroencephalic signal can be performed at the frequency of 125Hz/12 bit.

The data of the eight channels, possibly downstream of said filtering and amplification, are stored on a memory medium 112, for example a µSD (*Micro Secure Digital*) in real time, for example in the form of an exportable file, in particular in the format EDF (*European Data Format*), which is closed automatically when a command to finish the acquisition is received.

Preferably, the acquisition phase is controlled according to a predetermined duration.

The eight acquired traces found on the EDF file in the µSD card can be transferred to further means of the processing unit 102, for example on an electronic computer 113, in particular by means of a USB cable or by extracting the same µSD card 112 or by using a wireless communication via cloud, network or also as associated with the mobile device 111, in particular with an application thereof.

Using dedicated software, unit 102 can generate a string of stimulation commands. In this example, there are four stimulation channels (Stim₁₋₄).

As mentioned, the processing software can also be installed, for example in the form of an app, on a smart phone or tablet, for example the device 111 of Figure 1, in wireless communication with one or more of the other components of the system 100.

A preferred method for the operations performed by the processing unit 102 for the generation of stimulation signals starting from the analysis and processing of the acquired signals will now be presented.

The processing unit 102 is configured to identify an instant, or a time interval, of the subject falling asleep (step 503 in Figure 3).

In order to identify the moment in which the subject passes from the waking state to that of falling asleep, the acquired EEG trace can be analyzed and treated by performing a series of operations as indicated below.

The acquired continuous EEG signal, for example recorded during the course of the night, can be divided, i.e. segmented, into a plurality of segments, or periods, for example lasting 6 or 10 s, which represents a standard length range documented in literature. The analyzes which will be discussed later can always refer to an already segmented path.

Preferably, said identification is performed on the basis of one or more of the parameters listed below.
▪ Spectral Power Density of one or more of the acquired electroencephalographic signals. This function describes the distribution of signal power in the frequency domain.
▪ The calculation of the Spectral Power Density can for example be set with a Hamming window of 2 s with overlap of 50%, therefore of 1 s. Once the Spectral Power Density for each channel has been evaluated, it is possible to proceed either by evaluating each frequency bin individually, or by averaging those belonging to the same frequency band. In particular, the theta frequency band (5.00-7.50 Hz) and the alpha frequency band (8.00-11.50 Hz) appear to be the most responsive with respect to the ability to discriminate the waking phase from that of sleep, but the delta bands (2.00-4.50 Hz), sigma (typical of sleep for its 'spindles' 12.00-15.50 Hz), beta (16.00-24.50 Hz) can also be evaluated.
▪ Spectral coherence, that is the degree of similarity between the electroencephalographic sinusoidal signals acquired in correspondence of different brain areas, as a description of the functional connectivity between different brain areas.

It is possible to evaluate the fronto-posterior, latero-lateral, fronto-central coherence. Also in this situation, the more responsive bands appear theta and alpha but the delta, sigma, beta bands can also be considered.
- Entropy, that is the degree of complexity and predictability of the fluctuations in a time series defined by the acquired electroencephalographic signals.

Entropy can be evaluated for example by means of the *Approximate Entropy* (ApEn) parameter, whose algorithm is known (and implemented, for example, in Matlab^{®}). Entropy can be calculated on all EEG channels and for each signal period. **If** the Entropy value has changed with respect to that calculated in previous periods (where the number of previous periods is obviously parameterized) by a quantity greater than an established threshold, a check is carried out. That is, it is verified that the exceeding by the variation of the threshold is also true for a certain number of successive periods (also parametric). Therefore, having calculated the entropy averaged over the periods subsequent to the first detection of the significant variation, and verifying whether it is maintained, it can be said to have identified the time on the border between wakefulness and sleep (the time of falling asleep).

Once the time of falling asleep has been identified, the periods of the EEG trace are analyzed in the previous ten minutes and the topography, frequency, intensity and duration of the cerebral rhythms which, on an individual level, led to falling asleep are calculated (phase 504 in Figure 3). Then, the stimulation parameters are set in such a way that the stimulations delivered by the device induce the cerebral rhythms to behave similarly to those identified during the individual fall asleep (phase 505 in Figure 3). The analyzes described can be performed following an automated procedure and / or using artificial intelligence algorithms, machine learning, neural networks.

Preferably, the stimulation signals (step 506 of Figure 3) are of the tACS type (transcranial stimulation with alternating current) consisting of sinusoidal waves, preferably with a current intensity comprised in an interval of 0.1-5mA and preferably a variable frequency comprised in an interval of 0.10 ÷ 50.00Hz.

The processed stimulation signals are then transmitted, again through any known means of communication, including wireless, and possibly downstream of an AD conversion, to the components of the stimulation unit 103.

As mentioned, in specific embodiments the data relating to the acquired and / or processed signals can be shared / stored in cloud mode.

As mentioned above, an embodiment of a wearable brain electrostimulation device suitable for use in the system 100 is shown in Figures 2A-2D and generally denoted with 1.

The device 1 includes a main body 10 substantially in the form of a helmet, in turn carrying a plurality of arms each capable of extending longitudinally on the scalp substantially according to a sagittal plane of the subject. In the present example, the plurality of arms includes at least a pair of temporal, or outer arms, 11 and 12 and a pair of median arms, 13 and 14. Each of the latter is configured to stimulate the underlying central and frontal regions. The temporal arms 11 and 12 can act as a support or constraint to the subject's head and / or be used for stimulation, in particular parietal, or for acquisition, for example of muscle activity of the underlying regions. These arms 11-14 are frontally connected at a front structure 15, which advantageously also houses a local control unit 30. The opposite longitudinal end of each arm can instead be free, i.e. not connected with other arms or structures.

The device and/or its arms can be made entirely or in part of yielding and/or flexible material.

Preferably, the arms 11-14 are movable and/or extendable.

In particular, one or more arms 11-14 can provide elastic means, for example with spring, to adapt its grip to the conformation and anthropometry of the subject's head, ensuring correct adherence for the purposes of acquisition and / or stimulation operations.

Furthermore, one or more arms 11-14 can comprise means 40 for adjusting the relative longitudinal extension, preferably operable by means of a wheel manipulation element.

In the present example, the median arms 13 and 14 comprise means 50 for adjusting the relative trans-cranial position, preferably configured to allow rotation around an oblique axis lying substantially on a frontal plane, as exemplified by the arrows of Figure 2B.

Preferably, two electrode housings are provided in each arm 11-14, for example in the form of a multi-connector. Advantageously, the electrodes can be disconnectable from the structure of the device 1, i.e. removable.

One or both of the temporal arms 11 and 12 have a mastoid branch 111, 121 extending substantially orthogonal to a longitudinal direction of development of the relative arm so as to be arranged, in use, behind the ears of the subject.

One or both of the branches 111 and 121 are preferably configured to support the aforementioned one or more reference electrodes (or otherwise positioned on the top of the scalp), i.e. one or more extra-cephalic electrodes.

Furthermore, such branches 111, 121 can ensure a better seal on the head.

In the front structure 15 two inputs can be provided for the aforementioned bipolar acquisition channels.

The aforementioned local control unit 30 is configured to receive acquisition and stimulation commands from the units 101 and 103 or from specific components thereof and to actuate the electrodes arranged or available on said arms so as to pick up acquisition signals or deliver stimulation signals.

The unit 30 can include, or be connected to, one or more switches, one or more data inputs and one or more signalling devices arranged at the front structure 15.

Consistent with what has been explained in relation to the apparatus 100, the local control unit 30 is configured to perform a transcranial stimulation with alternating current tACS preferably based on signals consisting of sinusoidal waves, preferably with constant current intensity and with variable frequency.

Typically, the stimulation operates over a period of time of the order of ten minutes which represents the average duration of the time of falling asleep. In some cases it may be envisaged to repeat the stimulation one or more times.

It will be appreciated that the helmet device 1 has a modular, easily wearable, light and adjustable structure. The main body 10 can be covered in silicone rubber for greater comfort during wearing.

For greater stability, the structure of the main body 10 can be implemented with a chin guard or chin strap and / or with a component for joining the arms of the helmet at the rear.

Device 1 can also include a positioning system on a support base for easy charging, which does not require the search and insertion of cables and / or accessories for wireless charging.

The present invention has been described up to now with reference to preferred embodiments. It is to be understood that there may be other embodiments that pertain to the same inventive core, as defined by the scope of the claims set out below.

## Claims

1. A wearable device (1) for the acquisition of electroencephalographic signals and for personalized brain electrostimulation, configured to induce falling asleep in a person wearing it, which device (1) includes:
▪ a main body (10) substantially in the form of a helmet, in turn carrying a plurality of arms (11-14) each adapted to extend sagittally on the scalp, which plurality of arms includes at least a pair of temporal arms (11, 12) and a pair of median arms (13, 14) frontally connected (15); and
▪ a local control unit (30), configured to receive acquisition and stimulation commands from a remote unit (110) and to operate a plurality of acquisition and stimulation electrodes arranged, or configured to be arranged, on said arms (11-14).

2. The device (1) according to claim 1, wherein said local control unit (30) is arranged at a front housing (15) of said main body (10).

3. The device (1) according to claim 1 or 2, wherein one or more of said arms (11-14) comprises means (40) for adjusting its sagittal extension, preferably operable by means of a wheel-shaped manipulation element.

4. The device (1) according to any one of the preceding claims, wherein one or more of said arms (11-14) comprises means (50) for adjusting the relative trans-cranial position, preferably configured to allow rotation about a frontal axis.

5. The device (1) according to any one of the preceding claims, wherein one of, or both, the temporal arms (11, 12) have a mastoid branch (111, 121) extending substantially orthogonal to a longitudinal direction of prevailing development of the arm, which branch is preferably configured to support one or more reference electrodes.

6. The device (1) according to any one of the preceding claims, wherein said arms (11-14) have a free rear longitudinal end.

7. The device (1) according to any one of the preceding claims, wherein said local control unit (30) is configured to perform an alternating current transcranial stimulation (tACS), preferably based upon signals consisting of sine waves, preferably with variable current intensity and/or frequency.

8. The device (1) according to the preceding claim, wherein said current intensity varies in a range of about 0.1-5mA.

9. The device (1) according to claim 7 or 8, wherein said frequency is variable in a range of about 0.10 ÷ 50.00 Hz.

10. The device (1) according to any one of the preceding claims, wherein said local control unit is configured for communication with a mobile device, in particular a *tablet* or a *smart phone* (111).

11. The device (1) according to any one of the preceding claims, comprising means for acquiring an electroencephalic signal and/or means for electroencephalic stimulation.

12. The device (1) according to any one of the preceding claims, comprising housings for said acquisition and/or stimulation electrodes positioned on said arms (11-14) at frontal, central and parietal scalp areas.

13. A system (100) of brain electrostimulation configured to induce the falling asleep of a subject, which system (100) comprises a wearable device (1) according to any one of claims 1 to 12 and includes the following units, in operational data communication with each other:
▪ an acquisition unit (101) of electroencephalographic signals of said subject;
▪ a processing unit (102) of said acquired electroencephalographic signals, configured to output personalized electroencephalic stimulation signals of said subject;
▪ a stimulation unit (103), configured to control the plurality of stimulation electrodes, when applied at least at the frontal, central, parietal and temporal regions of the scalp of said subject, according to said stimulation signals.

14. The system (100) according to the preceding claim, wherein said processing unit (102) is configured to identify an instant, or a time interval, of falling asleep of the subject.

15. The system (100) according to the preceding claim, wherein said processing unit (102) is configured to identify an instant, or a time interval, of falling asleep of the subject based upon one or more of the following parameters: spectral power density of one or more of the electroencephalographic signals acquired by said acquisition unit (101); coherence, that is the degree of similarity between the electroencephalographic oscillatory signals acquired at different brain areas; entropy, that is the degree of complexity and predictability of the fluctuations in a time series defined by the acquired electroencephalographic signals.

16. The system (100) according to any one of claims 13 to 15, wherein said stimulation unit (103) is configured to perform transcranial alternating current stimulation (tACS), preferably based upon signals made of sine waves, preferably with variable current intensity and/or frequency.

17. The system (100) according to the previous claim, wherein said current intensity varies in a range of about 0.1-5mA.

18. The system (100) according to claim 16 or 17, wherein said frequency is variable in a range of about 0.10 ÷ 50.00 Hz.

19. The system (100) according to any of claims 13 to 18, which is configured for communication with a mobile device, in particular a *tablet* or *smart phone* (111).

## Patentansprüche

1. Tragbare Vorrichtung (1) für die Erfassung von elektroenzephalographischen Signalen und für eine personalisierte Gehirnelektrostimulation, die konfiguriert ist, um ein Einschlafen bei einer sie tragenden Person zu induzieren, wobei die Vorrichtung (1) einschließt:
▪ einen Grundkörper (10) im Wesentlichen in der Form eines Helms, der seinerseits eine Vielzahl von Armen (11-14) trägt, die jeweils angepasst sind, um sich sagittal auf der Kopfhaut zu erstrecken, wobei die Vielzahl von Armen mindestens ein Paar von Temporalarmen (11, 12) und ein Paar von Medianarmen (13, 14) einschließt, die frontal verbunden (15) sind; und
▪ eine lokale Steuereinheit (30), die konfiguriert ist, um Erfassungs- und Stimulationsbefehle von einer entfernten Einheit (110) zu empfangen und eine Vielzahl von Erfassungs- und Stimulationselektroden zu betreiben, die an den Armen (11-14) angeordnet sind oder dazu konfiguriert sind, daran angeordnet zu werden.

2. Vorrichtung (1) nach Anspruch 1, wobei die lokale Steuereinheit (30) an einem vorderen Gehäuse (15) des Grundkörpers (10) angeordnet ist.

3. Vorrichtung (1) nach Anspruch 1 oder 2, wobei einer oder mehrere der Arme (11-14) Mittel (40) zum Einstellen seiner sagittalen Ausdehnung umfasst, vorzugsweise betreibbar mittels eines radförmigen Manipulationselements.

4. Vorrichtung (1) nach einem der vorstehenden Ansprüche, wobei einer oder mehrere der Arme (11-14) Mittel (50) zum Einstellen der relativen transkraniellen Position umfasst, vorzugsweise konfiguriert, um eine Rotation um eine frontale Achse herum zu ermöglichen.

5. Vorrichtung (1) nach einem der vorstehenden Ansprüche, wobei einer der oder beide Temporalarme (11, 12) einen Mastoidzweig (111, 121) aufweisen, der sich im Wesentlichen orthogonal zu einer Längsrichtung der vorherrschenden Entwicklung des Arms erstreckt, wobei der Zweig vorzugsweise konfiguriert ist, um eine oder mehrere Referenzelektroden zu tragen.

6. Vorrichtung (1) nach einem der vorstehenden Ansprüche, wobei die Arme (11-14) ein freies hinteres Längsende aufweisen.

7. Vorrichtung (1) nach einem der vorstehenden Ansprüche, wobei die lokale Steuereinheit (30) konfiguriert ist, um eine transkranielle Wechselstromstimulation (tACS) durchzuführen, vorzugsweise basierend auf Signalen, die aus Sinuswellen bestehen, vorzugsweise mit variabler Stromstärke und/oder Frequenz.

8. Vorrichtung (1) nach dem vorstehenden Anspruch, wobei die Stromstärke in einem Bereich von etwa 0,1-5 mA variiert.

9. Vorrichtung (1) nach Anspruch 7 oder 8, wobei die Frequenz in einem Bereich von etwa 0,10 ÷ 50,00 Hz variabel ist.

10. Vorrichtung (1) nach einem der vorstehenden Ansprüche, wobei die lokale Steuereinheit für die Kommunikation mit einer mobilen Vorrichtung, insbesondere einem *Tablet* oder einem *Smartphone* (111), konfiguriert ist.

11. Vorrichtung (1) nach einem der vorstehenden Ansprüche, umfassend Mittel zum Erfassen eines elektroenzephalischen Signals und/oder Mittel für eine elektroenzephalische Stimulation.

12. Vorrichtung (1) nach einem der vorstehenden Ansprüche, umfassend Gehäuse für die Erfassungs- und/oder Stimulationselektroden, die an den Armen (11-14) in frontalen, zentralen und parietalen Kopfhautbereichen positioniert sind.

13. System (100) für eine Gehirnelektrostimulation, das konfiguriert ist, um das Einschlafen eines Subjekts zu induzieren, wobei das System (100) eine tragbare Vorrichtung (1) nach einem der Ansprüche 1 bis 12 umfasst und die folgenden Einheiten einschließt, die in betrieblicher Datenkommunikation miteinander stehen:
▪ eine Erfassungseinheit (101) für elektroenzephalographische Signale des Subjekts;
▪ eine Verarbeitungseinheit (102) der erfassten elektroenzephalographischen Signale, die konfiguriert ist, um personalisierte elektroenzephalische Stimulationssignale des Subjekts auszugeben;
▪ eine Stimulationseinheit (103), die konfiguriert ist, um die Vielzahl von Stimulationselektroden zu steuern, wenn sie mindestens an den frontalen, zentralen, parietalen und temporalen Regionen der Kopfhaut des Subjekts gemäß den Stimulationssignalen angebracht sind.

14. System (100) nach dem vorstehenden Anspruch, wobei die Verarbeitungseinheit (102) konfiguriert ist, um einen Zeitpunkt oder ein Zeitintervall des Einschlafens des Subjekts zu identifizieren.

15. System (100) nach dem vorstehenden Anspruch, wobei die Verarbeitungseinheit (102) konfiguriert ist, um einen Zeitpunkt oder ein Zeitintervall des Einschlafens des Subjekts basierend auf einem oder mehreren der folgenden Parameter zu identifizieren: spektrale Leistungsdichte eines oder mehrerer der elektroenzephalographischen Signale, die durch die Erfassungseinheit (101) erfasst werden; Kohärenz, was der Grad von Ähnlichkeit zwischen den elektroenzephalographischen oszillatorischen Signalen ist, die in unterschiedlichen Gehirnbereichen erfasst werden; Entropie, was der Grad von Komplexität und Vorhersagbarkeit der Schwankungen in einer Zeitreihe ist, die durch die erfassten elektroenzephalographischen Signale definiert ist.

16. System (100) nach einem der Ansprüche 13 bis 15, wobei die Stimulationseinheit (103) konfiguriert ist, um transkranielle Wechselstromstimulation (tACS) durchzuführen, vorzugsweise basierend auf Signalen, die aus Sinuswellen bestehen, vorzugsweise mit variabler Stromintensität und/oder Frequenz.

17. System (100) nach dem vorstehenden Anspruch, wobei die Stromstärke in einem Bereich von etwa 0,1-5 mA variiert.

18. System (100) nach Anspruch 16 oder 17, wobei die Frequenz in einem Bereich von etwa 0,10 ÷ 50,00 Hz variabel ist.

19. System (100) nach einem der Ansprüche 13 bis 18, das für die Kommunikation mit einer mobilen Vorrichtung, insbesondere einem *Tablet* oder *Smartphone* (111), konfiguriert ist.

## Revendications

1. Dispositif portable (1) pour l'acquisition de signaux électroencéphalographiques et pour l'électrostimulation cérébrale personnalisée, configuré pour induire l'endormissement chez une personne qui le porte, lequel dispositif (1) comporte :
▪ un corps principal (10) sensiblement sous la forme d'un casque, portant à son tour une pluralité de bras (11-14) chacun adapté pour s'étendre de manière sagittale sur le cuir chevelu, laquelle pluralité de bras comporte au moins une paire de bras temporaux (11, 12) et une paire de bras médians (13, 14) reliés frontalement (15) ; et
▪ une unité de commande locale (30), configurée pour recevoir des ordres d'acquisition et de stimulation à partir d'une unité à distance (110) et pour faire fonctionner une pluralité d'électrodes d'acquisition et de stimulation agencées, ou conçues pour être agencées, sur lesdits bras (11-14).

2. Dispositif (1) selon la revendication 1, dans lequel ladite unité de commande locale (30) est agencée au niveau d'un boîtier avant (15) dudit corps principal (10).

3. Dispositif (1) selon la revendication 1 ou 2, dans lequel un ou plusieurs desdits bras (11-14) comprennent un moyen (40) permettant d'ajuster son extension sagittale, de préférence actionnable au moyen d'un élément de manipulation en forme de roue.

4. Dispositif (1) selon l'une quelconque des revendications précédentes, dans lequel un ou plusieurs desdits bras (11-14) comprennent un moyen (50) permettant d'ajuster la position transcrânienne relative, de préférence conçu pour permettre une rotation autour d'un axe frontal.

5. Dispositif (1) selon l'une quelconque des revendications précédentes, dans lequel l'un des bras temporaux (11, 12), ou les deux, ont une branche mastoïdienne (111, 121) s'étendant sensiblement orthogonale à une direction longitudinale de développement dominant du bras, laquelle branche est de préférence conçue pour supporter une ou plusieurs électrodes de référence.

6. Dispositif (1) selon l'une quelconque des revendications précédentes, dans lequel lesdits bras (11-14) ont une extrémité longitudinale arrière libre.

7. Dispositif (1) selon l'une quelconque des revendications précédentes, dans lequel ladite unité de commande locale (30) est configurée pour effectuer une stimulation transcrânienne à courant alternatif (tACS), de préférence sur la base de signaux constitués d'ondes sinusoïdales, de préférence avec une intensité de courant et/ou une fréquence variables.

8. Dispositif (1) selon la revendication précédente, dans lequel ladite intensité de courant varie dans une plage d'environ 0,1 à 5 mA.

9. Dispositif (1) selon la revendication 7 ou 8, dans lequel ladite fréquence est variable dans une plage d'environ 0,10 ÷ 50,00 Hz.

10. Dispositif (1) selon l'une quelconque des revendications précédentes, dans lequel ladite unité de commande locale est configurée pour une communication avec un dispositif mobile, en particulier une *tablette* ou un *mobile multifonction* (111).

11. Dispositif (1) selon l'une quelconque des revendications précédentes, comprenant un moyen permettant d'acquérir un signal électroencéphalique et/ou un moyen pour une stimulation électroencéphalique.

12. Dispositif (1) selon l'une quelconque des revendications précédentes, comprenant des boîtiers pour lesdites électrodes d'acquisition et/ou de stimulation positionnées sur lesdits bras (11-14) au niveau des zones frontales, centrales et pariétales du cuir chevelu.

13. Système (100) d'électrostimulation cérébrale configuré pour induire l'endormissement d'un sujet, lequel système (100) comprend un dispositif portable (1) selon l'une quelconque des revendications 1 à 12 et comporte les unités suivantes, en communication de données fonctionnelles l'une avec l'autre :
▪ une unité d'acquisition (101) de signaux électroencéphalographiques dudit sujet ;
▪ une unité de traitement (102) desdits signaux électroencéphalographiques acquis, configurée pour émettre des signaux de stimulation électroencéphalique personnalisés dudit sujet ;
▪ une unité de stimulation (103), configurée pour commander la pluralité d'électrodes de stimulation, lorsqu'elles sont appliquées au moins au niveau des régions frontale, centrale, pariétale et temporale du cuir chevelu dudit sujet, selon lesdits signaux de stimulation.

14. Système (100) selon la revendication précédente, dans lequel ladite unité de traitement (102) est configurée pour identifier un instant, ou un intervalle de temps, d'endormissement du sujet.

15. Système (100) selon la revendication précédente, dans lequel ladite unité de traitement (102) est configurée pour identifier un instant, ou un intervalle de temps, d'endormissement du sujet sur la base d'un ou plusieurs des paramètres suivants : densité de puissance spectrale d'un ou plusieurs des signaux électroencéphalographiques acquis par ladite unité d'acquisition (101) ; cohérence, c'est-à-dire le degré de similitude entre les signaux oscillatoires électroencéphalographiques acquis au niveau des différentes zones du cerveau ; entropie, c'est-à-dire le degré de complexité et de prévisibilité des fluctuations dans une série temporelle définie par les signaux électroencéphalographiques acquis.

16. Système (100) selon l'une quelconque des revendications 13 à 15, dans lequel ladite unité de stimulation (103) est configurée pour effectuer une stimulation transcrânienne à courant alternatif (tACS), de préférence basée sur des signaux constitués d'ondes sinusoïdales, de préférence avec une intensité de courant et/ou une fréquence variables.

17. Système (100) selon la revendication précédente, dans lequel ladite intensité de courant varie dans une plage d'environ 0,1 à 5 mA.

18. Système (100) selon la revendication 16 ou 17, dans lequel ladite fréquence est variable dans une plage d'environ 0,10 ÷ 50,00 Hz.

19. Système (100) selon l'une quelconque des revendications 13 à 18, qui est configuré pour une communication avec un dispositif mobile, en particulier une *tablette* ou un *mobile multifonction* (111).
